# EUROPÄISCHE PATENTSCHRIFT

(11) **EP 0 301 485 B1**
(45) Veröffentlichungstag und Bekanntmachung des Hinweises auf die Patenterteilung: **15.12.1993**
(21) Anmeldenummer: 88112059.6
(22) Anmeldetag: 26.07.1988
(51) Int. Cl.: C12N 15/74, C12N 15/62, C12P 21/02, C12N 15/00

(54) **Expressionsvektoren und Verfahren unter deren Verwendung zur Gewinnung von Cro/beta-Galaktosidase/PTH-Fusionsproteinen und von PTH**
Expression vectors and processes using same in the production of cro/beta-galactosidase/PTH fusion proteins and of PTH
Vecteurs d'expression et procédé les utilisant pour la production de protéines de fusion de cro/bêta-galactosidase/PTH et de PTH

(30) Priorität: 30.07.1987 DE 3725320
(43) Veröffentlichungstag der Anmeldung: 01.02.1989
(73) Patentinhaber: Gesellschaft für Biotechnologische Forschung mbH (GBF), D-38124 Braunschweig (DE)
(72) Erfinder: Wingender, Edgar, Dr., D-3300 Braunschweig (DE); Mayer, Hubert, Dr., D-3300 Braunschweig (DE); Schlüter, Klaus-Dieter, D-3300 Braunschweig (DE); Morelle, Gilles, Dr., D-3300 Braunschweig (DE); Mielke, Heiko, Dr., D-3300 Braunschweig (DE); Blöcker, Helmut, Dr., D-3300 Braunschweig (DE); Frank, Ronald, Dr., D-3300 Braunschweig (DE)
(74) Vertreter: Boeters, Hans Dietrich, Dr.

(56) Entgegenhaltungen:
- EP-A- 0 139 076

## Beschreibung

Parathormon (PTH) reguliert den Calciumspiegel des Blutplasmas, indem es u.a. auf- und abbauende Wirkungen auf das Skelett entfaltet aber auch die Calciumrückresorption durch die Niere reguliert. Es gibt Anhaltspunkte dafür, daß der knochenaufbauende Effekt des PTH zur klinischen Behandlung von Osteoporose ausgenutzt werden kann; J. Bone Mineral Res., 1 (1987) 377-382 (Slovik et al.).

Zur Bereitstellung der dafür erforderlichen Mengen an Parathormon bietet sich dessen Expression in E. coli an, da PTH zu seiner Wirksamkeit nicht auf posttranslationale Modifizierungen angewiesen ist. Die direkte Expression von PTH in E. coli ist bekannt; vgl. beispielsweise DE-A-33 12 928 Diese direkte Expression erwies sich jedoch noch als verbesserungsbedürftig.

Aufgabe der Erfindung ist es, Vektoren zur Expression von Cro/β-Galaktosidase/PTH-Fusionsproteinen sowie Verfahren zur Gewinnung dieser Fusionsproteine und von PTH vorzusehen.

Zur Lösung dieser Aufgabe sieht man erfindungsgemäß einen Expressionsvektor für E. coli mit (in Transkriptionsrichtung)
- Antibiotikumresistenz-Gen,
- Gen eines Hybridproteins und
- Gen eines gewünschten Proteins vor, wobei der
Expressionsvektor dadurch gekennzeichnet ist, daß es sich um pEX2 mit folgenden Modifikationen handelt:
- eine Deletion minimal im Bereich von der EcoRV- bis zur XbaI-Schnittstelle bis maximal im Bereich von der HpaI-Schnittstelle (stromabwärts in Nachbarschaft zur HindIII-Schnittstelle) bis zur XbaI-Schnittstelle und
- Insertion des PTH-Gens (als Gen des gewünschten Proteins) vor der XbaI-Schnittstelle.

Für den pEX2-Vektor kann auf die Hinterlegung NCIB 12319 sowie DE-A-3 619 902 verwiesen werden.

Mit Expressionsvektoren, die die erfindungsgemäßen Merkmale aufweisen, läßt sich beispielsweise verglichen mit einem Einsatz eines unmodifizierten pEX2-Vektors eine ausgezeichnete Ausbeute erreichen.

Weitere Lösungen und Ausführungsformen der Erfindung ergeben sich aus den Ansprüchen 2 bis 12.

Nachstehend wird die Erfindung näher erläutert. Es zeigen:
Figuren 1A1 bis 1A3 erfindungsgemäße Expressionsvektoren;
Figuren 1B1 bis 1B3 Teil-DNA-Sequenzen erfindungsgemäß erhaltener Cro/β-Galaktosidase/PTH-Fusionsproteine;
Figuren 2A und 2B Gelelektrophoresen zur Erläuterung der Expression von Cro/β-Galaktosidase/PTH-Fusionsproteinen;
Figuren 3A und 3B graphische Darstellungen zur Erläuterung der Optimierung von Wachstums- und Induktionsdauer; und
Figur 4 Gelelektrophoresen zur Erläuterung der Hydrolyse von Fusionsproteinen und der Hydrolysatreinigung.

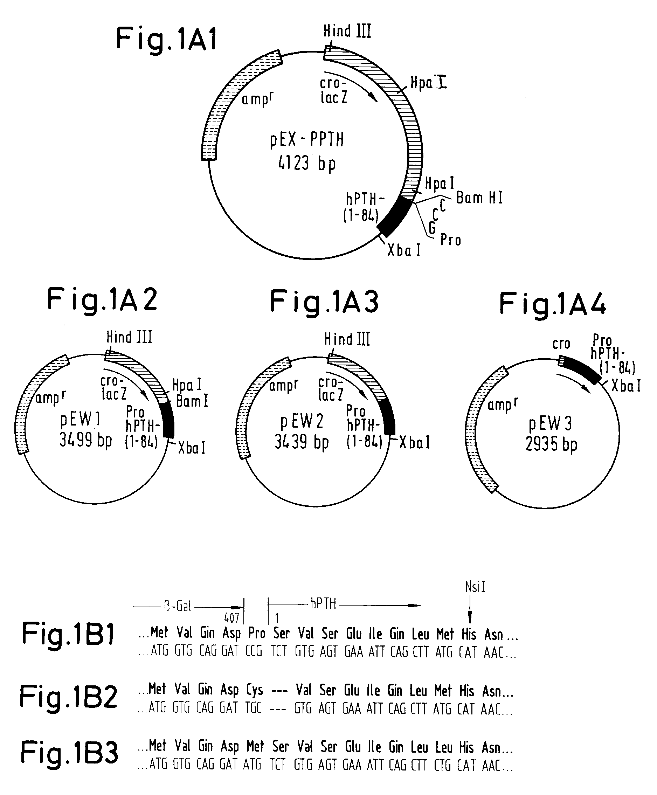

Die Figuren 1A1 bis 1A4 zeigen Expressionsvektoren für menschliches Parathormon (hPTH). Gemäß Figur 1A1 ist an das Gen für die Aminosäuren 1 bis 407 eines Cro/β-Galaktosidase-Hybridproteins (dunkel schraffiert) das hPTH-Gen über ein Prolin-Kodon fusioniert (schwarz). Die Transkription erfolgt in der angegebenen Richtung (Pfeil) unter der Kontrolle des P_{R}-Promotors des lambda-Phagen (weiß). Aus diesem Konstrukt wurden der HpaI/HpaI-Bereich (pEW 1; Figur 1A2), der HpaI/ BamHI-Bereich (pEW 2; Figur 1A3) oder der HindIII/BamHI-Bereich (pEW 3 für nicht-erfindungsgemäßen Vergleiche; Figur 1A4) deletiert.

Gemäß Figur 1B1 erfolgte die Fusion über ein Prolin-Kodon (Figuren 1A1 bis 1A4), gemäß Figur 1B2 über ein Cystein-Kodon und gemäß Figur 1B3 über ein Methionin-Kodon. Diese Sequenzen sowie die nachfolgenden Sequenzen bis zur (ursprünglichen) NsiI-Stelle wurden chemisch synthetisiert (kursiv).

### Expression

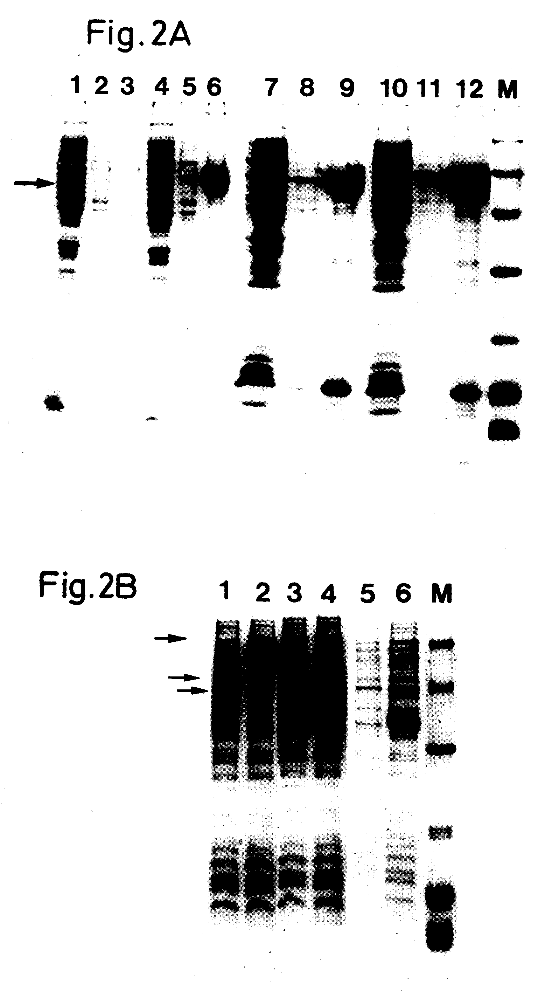

Mit einem Konstrukt gemäß Anspruch 4 (pEX-PPTH; Figuren 1A1 und 1B1) transformierte E.-coli-Zellen (N 4830), die über ein lambda-Repressor-Gen verfügen (J. Mol. Biol., 140 (1980) 57-75; Gottesman et al.) wurden kultiviert. Die Sequenz war gemäß Proc. Natl. Acad. Sci. USA, 74 (1977) 5463-5467 (Sänger et al.) verifiziert worden. Durch Temperaturerhöhung von 30 auf 42 °C fand Induktion statt und wurde Fusionsprotein gebildet (vgl. etwa Figur 2 jeweils Bahnen 3 und 6). Das Fusionsprotein wurde in den E.-coli-Zellen in Form von Einschlußkörperchen abgelagert, wie durch elektronenmikroskopische Untersuchungen nachgewiesen wurde. Diese Einschlußkörperchen ließen sich durch selektive Harnstoffextraktion stark anreichern (vgl. Figur 2 jeweils Bahnen 4 bis 6).

Entsprechende Expressionen wurden mit Konstrukten gemäß Anspruch 5 (Figur 1B2) und Anspruch 6 (Figur 1B3) sowie gemäß Anspruch 4 (Konstrukte pEW 1 und pEW 2; Figuren 1A2, 1A3 und 1B1) bzw. mit den damit transformierten E.-coli-Zellen durchgeführt (Figur 2a Bahnen 7 bis 12 und Figur 2b).

Figur 2A: Mit dem Konstrukt gemäß Anspruch 4 (pEX-PPTH; Figuren 1A1, 1B1) transformierte E.-coli-Zellen wurden unter nicht-induzierenden Bedingungen (Bahnen 1 bis 3) oder unter induzierenden Bedingungen (Bahnen 4 bis 6) kultiviert, geerntet, aufgeschlossen und nacheinander mit 0 M (Bahnen 1 und 4), 2 M (Bahnen 2 und 5) und 9 M Harnstoff extrahiert (Bahnen 3 und 6). Gezeigt ist ein SDS-Polyacrylamid-Gel der erhaltenen Extrakte nach Amidoschwarz-Färbung. Bahnen 7 bis 9: Wie Bahnen 4 bis 6, jedoch mit Konstrukt gemäß Anspruch 5 (Figur 1B2); Bahnen 10 bis 12: Wie Bahnen 4 bis 6, jedoch mit Konstrukt gemäß Anspruch 6 (Figur 1B3).

Markerproteine (Bahn M) waren β-Galaktosidase (100 kDa), Rinderserumalbumin (67 kDa), Ovalbumin (45 kDa), Carboanhydrase (29 kDa), Trypsininhibitor aus Sojabohnen (21 kDa), Cytochrom-C-Trypsininhibitor aus der Lunge (6,5 kDa).

Figur 2B (Expression der verkürzten Fusionsproteine): E.-coli-Kulturen mit Konstrukten gemäß Anspruch 4 (pEX-PPTH: Bahnen 1 und 2; pEW 1: Bahnen 3 und 4; pEW 2: Bahnen 5 und 6) wurden unter nicht-induzierenden Bedingungen (Bahnen 1, 3 und 5) oder induzierenden Bedingungen (Bahnen 2, 4 und 6) aufgezogen, geerntet und mit SDS-Puffer aufgeschlossen. Die auf diese Weise erhaltenen Gesamtzellextrakte wurden gelelektrophoretisch analysiert.
Markerproteine (Bahn M) vgl. Figur 2A.

### Optimierung der Wachstums- und Induktionsdauer

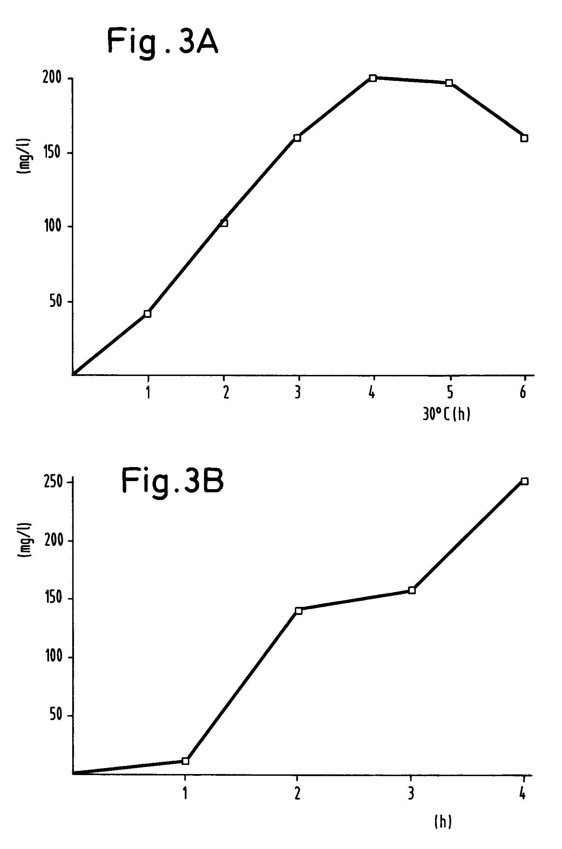

Am Beispiel des Konstruktes gemaß Anspruch 4 (pEX-PPTH; Figuren 1A1 und 1B1) wurde versucht, die Wachstumsbedingungen der Expressionskulturen zu optimieren, wobei die Menge an gebildetem Fusionsprotein in einem 9 M Harnstoffextrakt als Kriterium diente. Eine Variation der Wachstumsdauer bei 30 °C mit nachfolgender 4-stündiger Induktion bei 42 °C ergab ein Optimum bei 4 bis 5 h (Figur 3A). In einem separaten Experiment (Figur 3B) wurde eine Induktionsdauer von 4 h als optimal ermittelt; Verlängerungen führten zu keiner weiteren Steigerung der Ausbeute (bis 21 h, nicht gezeigt), sondern oberhalb 21 h wieder zu einer Abnahme der Menge an Fusionsprotein.

Es wurden reproduzierbar 250 mg Fusionsprotein/l Kulturmedium erhalten. Diese Menge macht 50 bis 60 % des Gesamtproteins aus und entspricht 42 mg hPTH/l. Mitunter wurden sogar noch deutlich höhere Ausbeuten erzielt (bis zu 400 mg Fusionsprotein/l).

Die Ausbeuten an Fusionsproteinen, die mit Konstrukten gemäß Anspruch 4 (pEW 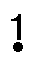 und pEW 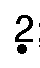 ; Figuren 1A2, 1A3 und 1B1) erhalten wurden, waren etwas geringer; die Ausbeute an spezifischer hPTH-Sequenz war jeweils vergleichbar mit derjenigen des Konstrukts gemäß Anspruch 4 (pEX-PPTH; Figuren 1A1 und 1B1).

Deutlich geringer war die Expression mit dem Konstrukt gemäß Anspruch 4 (pEW 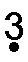 ; Figuren 1A4 und 1B1).

Figur 3A: E.-coli-Zellen, die mit einem Konstrukt gemäß Anspruch 4 (pEX-PPTH; Figuren 1A1 und 1B1) transformiert worden waren, wurden in 1 l LB-Medium die angegebenen Zeiten geschüttelt, bei 42 °C 4 h lang induziert, geerntet und aufgearbeitet (vgl. Figur 2). Die Menge an gebildetem Fusionsprotein im 9 M Harnstoff-Extrakt wurde gemäß Anal. Biochem., 72 (1976) 245-248 (Bradford) gemessen.

Figur 3B: Nach 3-stündigem Wachstum bei 30 °C wurde die Induktionsdauer bei 42 °C variiert; im übrigen wurde gemäß den Angaben zu Figur 3A verfahren.

### Spaltung der Fusionsproteine

Fusionsproteine aus Expressionsvektoren gemäß Anspruch 4 (vgl. Figuren 1A1 bis 1A4 und 1B1; Konstrukte pEX-PPTH, pEW 1, pEW 2 und pEW 3). Durch die Einfügung des Prolin-Kodons zur Ausbildung einer Asp-Pro-Verknüpfung des Cro/β-Galaktosidase-Teiles mit dem PTH-Anteil wird eine besondere Säurelabilität erreicht; vgl. Methods Enzymol., XLVII (1977) 145 ff. (Landon et al.). Durch Säurehydrolyse gemäß beispielsweise Biochem. Biophys. Res. Comm., 40 (1970) 1173-1178 (Piskiewicz et al.) wird freies Hormon erzeugt, das N-terminal um einen Prolinrest erweitert ist. Es ist überraschend, daß durch diese Säurehydrolyse die Amidgruppe des Asparagins nicht angegriffen wird. Sofern bzw. da PTH selbst keine Asp-Pro-Kombination enthält, kann der N-terminale Prolinrest durch Edman-Abbau oder mit Hilfe einer Iminopeptidase entfernt werden.

Fusionsproteine aus Expressionsvektoren gemäß Anspruch 5 (vgl. Figur 1B2). Die Substition des Serins(1) durch Cystein eröffnet zwei Wege zur chemischen Spaltung des resultierenden Fusionsproteins.
(a) Man kann das Cystein mit Ellman's Reagens zum Disulfid oxidieren und danach mit Kaliumcyanid spalten, so daß ein zum Iminothiazolidin zyklisierter Cysteinrest den N-Terminus des freigesetzten Produktes bildet; vgl. Methods Enzymol., XLVII (1977) 129 ff. (Stark et al.).
(b) Alternativ kann man nach reversiblem Schutz der internen Methioninreste (etwa durch selektives Oxidieren) das Cystein alkylieren und hier anschließend mit Bromcyan spalten, wobei der Cysteinrest wieder zum natürlichen Serinrest umgewandelt wird; vgl. BBRC, 17 (1964) 709-715 (Awad & Wilcox).

Fusionsproteine aus Expressionsvektoren gemäß Anspruch 6 (vgl. Figur 1B3). Die Einfügung des Methioninrestes bei gleichzeitiger Substitution der internen Methionine dient der Abspaltung des PTH aus dem Fusionsprotein mit Bromcyan; vgl. J. Biol. Chem., 237 (1962) 1856-1860 (Gross & Witkop). Der Austausch von Met(8) und Met(18) durch Leucin läßt nach gängigen Vorhersagemodellen für Protein-Sekundärstrukturen die geringste Veränderung in der Proteinfaltung erwarten. Außerdem ist dieser Ersatz auch in der Natur zu beobachten, etwa bei pPTH in Position 18; vgl. Biochemistry, 13 (1984) 1994-1999 (Sauer et al.).

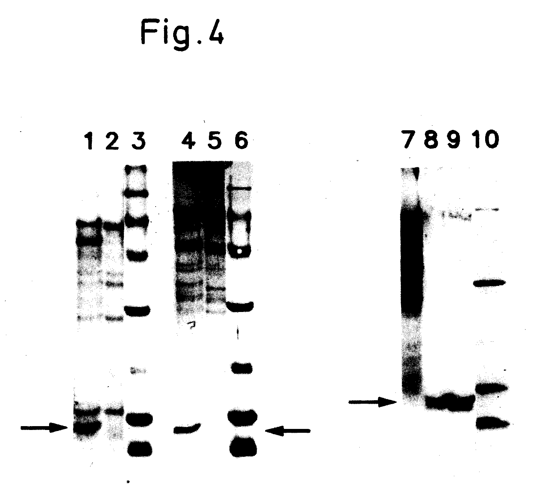

In einem Beispiel wurde das Fusionsprotein aus einem Konstrukt gemäß Anspruch 4 (pEX-PPTH; Figuren 1A1 und 1B1) mit 9 M Harnstoff extrahiert. Der Harnstoffextrakt mit ca. 15 mg Fusionsprotein/ml wurde direkt mit dem gleichen Volumen konzentrierter Ameisensäure versetzt und 5 bis 6 Tage bei 37 °C gehalten. Gelektrophoretische Analyse des Hydrolysats zeigte als eine der prominenten Banden eine mit dem hPTH-Molekulargewicht (9500 kDa, Figur 4 Bahn 1), die mit Anti-PTH-Antikörpern reagierte (Figur 4 Bahn 4). Diese Bande erschien nicht im Hydrolysat des Fusionsproteins, das mit einem Konstrukt gemäß Anspruch 5 (Figur 1B2) erhalten wurde und keine säurelabile Spaltstelle zwischen dem Cro/β-Galaktosidase- und dem hPTH-Anteil besaß (Figur 4 Bahnen 2 und 5).

Niedrigere Konzentrationen an Ameisensäure verlängerten die Hydrolysedauer erheblich. Niedrigere Konzentrationen an Harnstoff verminderten die Ausbeute an freiem Pro(-1)-PTH. Höhere Temperaturen führten zum Abbau auch von PTH (bis 95 °C getestet). Ohne Einfluß war die Proteinkonzentration.

Das Säurehydrolysat wurde ausgiebig gegen 10 mM Ammoniumacetat dialysiert und auf eine 8 M Harnstoff-Konzentration eingestellt. Das Dialysat wurde danach über Carboxymethylcellulose aufgetrennt, die mit dem gleichen Puffer äquilibriert worden war; Biochemistry, 13 (1974) 1646-1652 (Keutmann et al.). Dazu konnte erfindungsgemäß (abweichend vom Stand der Technik) überraschend mit einem Stufengradienten eluiert werden; 50 mM Ammoniumacetat und 8 M Harnstoff. Die Reinheit des Materials wurde durch elektrophoretische Analyse (Figur 4 Bahnen 7 bis 9) über zwei verschiedene Gelsysteme bewiesen (SDS-Polyacrylamid und Essigsäure/Harnstoff-Polyacrylamid).

Das gereinigte Pro(-1 )-hPTH wurde gegen 50 mM Essigsäure dialysiert und portioniert bei -20 °C gelagert. Die Sequenzierung ergab einen korrekten N-Terminus einschließlich Gln(6) und Asn(10).

Figur 4 (Hydrolyse des Fusionsproteins und Reinigung von Pro(-1)-hPTH): Das mit Hilfe des Konstrukts gemäß Anspruch 4 (pEX-PPTH, Figuren 1A1 und 1B1; Bahn 1) bzw. mit Hilfe des Konstrukts gemäß Anspruch 5 (Figur 1B2; Bahn 2) wurde gegen 10 mM Ammoniumacetat dialysiert und einer SDS-Polyacrylamid-Gelelektrophorese unterzogen. Das Gel wurde in einem Western-Blot-Test mit einem Anti-PTH-Antikörper und Peroxydase-Färbung analysiert (Bahnen 4 und 5). Die Markerproteine waren dieselben wie bei Figur 2; Bahn 3: SDS-Polyacrylamid-Gel; Bahn 6: Mit Amidoschwarz gefärbtes Nitrocellulose-Filter.

Das saure Hydrolysat wurde auf eine 8 M Harnstoff-Konzentration eingestellt und auf Carboxymethylcellulose aufgetragen. Nach dem Auswaschen des nichtbindenden Anteils (Bahn 7) wurde mit einer 50 mM Ammoniumacetat-Stufe eluiert (Bahnen 8 und 9; zwei Fraktionen aus dieser Stufe). Die Markerproteine waren dieselben.

Sofern mit den erfindungsgemäßen Expressionsvektoren PTH-Modifikationen gewinnbar sind, vergleiche man EP-A-301484 bezüglich der vorteilhaften Eigenschaften.

## Patentansprüche

1. Expressionsvektor für E. coli mit (in Transkriptionsrichtung)
-- Antibiotikumresistenz-Gen,
-- Gen eines Hybridproteins und
-- Gen eines gewünschten Proteins, dadurch ***gekennzeichnet,*** daß es sich bei dem Expressionsvektor um pEX2 mit folgenden Modifikationen handelt:
-- eine Deletion minimal im Bereich von der EcoRV- bis zur XbaI-Schnittstelle bis maximal im Bereich von der HpaI-Schnittstelle (stromabwärts in Nachbarschaft zur HindIII-Schnittstelle) bis zur XbaI-Schnittstelle und
-- Insertion des PTH-Gens (als Gen des gewünschten Proteins) vor der XbaI-Schnittstelle.

2. Expressionsvektor nach Anspruch 1, dadurch **gekennzeichnet,** daß das Ile-Kodon ATA des PTH-Gens durch ATT ersetzt ist.

3. Expressionsvektor nach einem der vorhergehenden Ansprüche, **gekennzeichnet** durch
(a) ein zusätzliches Pro-Kodon vor der PTH kodierenden DNA-Sequenz oder
(b) ein Cys-Kodon anstelle des Ser(1)-Kodons des PTH-Gens oder
(c) ein zusätzliches Met-Kodon vor der PTH kodierenden DNA-Sequenz und 2 Leu-Kodons anstelle des Met(8)- und des Met(18)-Kodons des PTH-Gens.

4. Expressionsvektor nach Anspruch 1 mit folgenden Merkmalen:
(a) P_{R}-Promotor (Fig. 1A1);
(b) gegebenenfalls Deletion des DNA-Bereichs zwischen den beiden HpaI-Schnittstellen (Fig. 1A1), die beide im Leseraster und hinter dem Kodon für Aminosäure 178 bzw. für Aminosäure 386 des Cro/β-Galaktosidase-Proteins (lacZ-Gen) liegen;
(c) EcoRV-Schnittstelle unmittelbar hinter dem Kodon für Aminosäure 407 (Asp) des Cro/β-Galaktosidase-Proteins (lacZ-Gen);
(d1) 5'-seitig in die EcoRV-Schnittstelle gemäß (c) eingesetztes PTH-Gen (Fig. 1A1) mit folgenden Merkmalen:
(d2) das Ile-Kodon ATA ist gegebenenfalls durch ATT ersetzt (Fig. 1B);
(d3) die DNA-Sequenz bis zur NsiI-Schnittstelle des PTH-Gens (Fig. B) ist chemisch synthetisiert;
(d4) der Rest des PTH-Gens (bis zur XbaI-Schnittstelle; Fig. 1A1) entstammt einem PTH-Gen, das aus einer genomischen Cosmid-Gen-Bank isoliert wurde, wobei dieser Rest 3'-seitig in die XbaI-Schnittstelle eingesetzt ist; und ferner
(d5) ein zusätzliches Pro-Kodon (Fig. 1B1) vor der PTH kodierenden DNA-Sequenz; und
(d6) eine neue BamHI-Schnittstelle (Fig. 1A1 und 1A2), die an der vormaligen EcoRV-Schnittstelle gemäß (c) (bzw. Fusionsstelle) erzeugt worden ist und gegebenenfalls aufgefüllt worden ist (Fig. 1A1), wobei die DNA-Sequenz (228 Aminosäuren) zwischen der ersten HpaI-Schnittstelle (vgl. (b); Fig. 1A1) und der BamHI-Schnittstelle (Figuren 1A1 und 1A3) deletiert worden ist.

5. Expressionsvektor nach Anspruch 1 mit folgenden Merkmalen:
(a) P_{R}-Promotor (Fig. 1A1);
(b) gegebenenfalls Deletion des DNA-Bereichs zwischen den beiden HpaI-Schnittstellen (Fig. 1A1), die beide im Leseraster und hinter dem Kodon für Aminosäure 178 bzw. für Aminosäure 386 des Cro/β-Galaktosidase-Proteins (lacZ-Gen) liegen;
(c) EcoRV-Schnittstelle unmittelbar hinter dem Kodon für Aminosäure 407 (Asp) des Cro/β-Galaktosidase-Proteins (lacZ-Gen);
(d1) 5'-seitig in die EcoRV-Schnittstelle gemäß (c) eingesetztes PTH-Gen (Fig. 1A1) mit folgenden Merkmalen:
(d2) das Ile-Kodon ATA ist gegebenenfalls durch ATT ersetzt (Fig. 1B);
(d3) die DNA-Sequenz bis zur NsiI-Schnittstelle des PTH-Gens (Fig. B) ist chemisch synthetisiert;
(d4) der Rest des PTH-Gens (bis zur XbaI-Schnittstelle; Fig. 1A1) entstammt einem PTH-Gen, das aus einer genomischen Cosmid-Gen-Bank isoliert wurde, wobei dieser Rest 3'-seitig in die XbaI-Schnittstelle eingesetzt ist; und ferner
(d5) ein Cys-Kodon anstelle des Ser(1)-Kodons des PTH-Gens (Fig. 1B2).

6. Expressionsvektor nach Anspruch 1 mit folgenden Merkmalen:
(a) P_{R}-Promotor (Fig. 1A1);
(b) gegebenenfalls Deletion des DNA-Bereichs zwischen den beiden HpaI-Schnittstellen (Fig. 1A1), die beide im Leseraster und hinter dem Kodon für Aminosäure 178 bzw. für Aminosäure 386 des Cro/β-Galaktosidase-Proteins (lacZ-Gen) liegen;
(c) EcoRV-Schnittstelle unmittelbar hinter dem Kodon für Aminosäure 407 (Asp) des Cro/β-Galaktosidase-Proteins (lacZ-Gen);
(d1) 5'-seitig in die EcoRV-Schnittstelle gemäß (c) eingesetztes PTH-Gen (Fig. 1A1) mit folgenden Merkmalen:
(d2) das Ile-Kodon ATA ist gegebenenfalls durch ATT ersetzt (Fig. 1B);
(d3) die DNA-Sequenz bis zur NsiI-Schnittstelle des PTH-Gens (Fig. B) ist chemisch synthetisiert;
(d4) der Rest des PTH-Gens (bis zur XbaI-Schnittstelle; Fig. 1A1) entstammt einem PTH-Gen, das aus einer genomischen Cosmid-Gen-Bank isoliert wurde, wobei dieser Rest 3'-seitig in die XbaI-Schnittstelle eingesetzt ist; und ferner
(d5) ein zusätzliches Met-Kodon (Fig. 1B3) vor der PTH kodierenden DNA-Sequenz; und
(d6) zwei Leu-Kodons anstelle des Met(8)- und des Met(18)-Kodons des PTH-Gens (Fig. 1B3).

7. Expressionsvektor nach einem der vorhergehenden Ansprüche, **gekennzeichnet** durch das pPTH-, bPTH- oder hPTH-Gen als PTH-Gen.

8. Verfahren zur Gewinnung eines Cro/β-Galaktosidase/PTH-Fusionsproteins, dadurch **gezeichnet,** daß man das Fusionsprotein mit Hilfe eines Expressionsvektors gemäß einem der vorhergehenden Ansprüche in E. coli exprimiert, extrahiert und gegebenenfalls isoliert.

9. Verfahren zur Gewinnung von PTH, dadurch **gekennzeichnet,** daß man mit Hilfe des Expressionsvektors gemäß Anspruch 3 (a) oder 4 in E. coli ein Cro/β-Galaktosidase/PTH-Fusionsprotein exprimiert und das auf diese Weise und durch Extraktion gewonnene Fusionsprotein sauer spaltet, den N-terminalen Prolinrest entfernt und PTH in an sich bekannter Weise reinigt und gewinnt oder
daß man das gemäß Anspruch 8 mit Hilfe des Expressionsvektors gemäß Anspruch 3 (a) oder 4 und durch Extraktion gewonnene Fusionsprotein sauer spaltet, den N-terminalen Prolinrest entfernt und PTH in an sich bekannter Weise reinigt und gewinnt.

10. Verfahren zur Gewinnung von PTH, dadurch **gekennzeichnet,** daß man mit Hilfe eines Expressionsvektors gemäß Anspruch 3 (b) oder 5 in E. coli ein Cro/β-Galaktosidase/PTH-Fusionsprotein exprimiert und das auf diese Weise und durch Extraktion gewonnene Fusionsprotein
(a) zum Disulfid oxidiert und mit Cyanid spaltet, wobei N-terminal ein zyklisierter Cysteinrest entsteht, oder
(b) nach reversiblem Schutz der Methioninreste und Alkylierung des Cys mit Bromcyan spaltet, wobei der Cysteinrest zum natürlichen Serinrest umgewandelt wird,
und PTH in an sich bekannter Weise reinigt und gewinnt oder daß man
das gemäß Anspruch 8 mit Hilfe des Expressionsvektors gemäß Anspruch 3 (b) oder 5 und durch Extraktion gewonnene Fusionsprotein
(a) zum Disulfid oxidiert und mit Cyanid spaltet, wobei N-terminal ein zyklisierter Cysteinrest entsteht, oder
(b) nach reversiblem Schutz der Methioninreste und Alkylierung des Cys mit Bromcyan spaltet, wobei der Cysteinrest zum natürlichen Serinrest umgewandelt wird,
und PTH in an sich bekannter Weise reinigt und gewinnt.

11. Verfahren zur Gewinnung von PTH, dadurch **gekennzeichnet,** daß man mit Hilfe des Expressionsvektors gemäß Anspruch 3 (c) oder 6 in E. coli ein Cro/β-Galaktosidase/PTH-Fusionsprotein exprimiert und aus dem auf diese Weise und durch Extraktion gewonnenen Fusionsprotein PTH mit Bromcyan abspaltet, in an sich bekannter Weise reinigt und gewinnt oder
daß man aus dem gemäß Anspruch 8 mit Hilfe des Expressionsvektors gemäß Anspruch 3 (c) oder 6 und durch Extraktion gewonnenen Fusionsprotein PTH mit Bromcyan abspaltet, in an sich bekannter Weise reinigt und gewinnt.

12. Verfahren nach Anspruch 9, 10 oder 11, dadurch **gekennzeichnet,** daß man das freigesetzte PTH als wässerige gepufferte harnstoffhaltige Lösung mit einem Stufengradienten an Carboxymethylcellulose reinigt.

## Claims

1. An expression vector for E. coli having (in the direction of transcription)
- antibiotic resistance gene,
- gene of a hybrid protein, and
- gene of a desired protein,
characterised in that the expression vector is pEX2 with the following modifications:
- a deletion that is at minimum in the region from the EcoRV cleavage site to the XbaI cleavage site to at maximum in the region from the HpaI cleavage site (downstream in the region of the HindIII cleavage site) to the XbaI cleavage site; and
- insertion of the PTH gene (as the gene of the desired protein) upstream of the XbaI cleavage site.

2. An expression vector according to claim 1, characterised in that the Ile codon ATA of the PTH gene has been replaced by ATT.

3. An expression vector according to any one of the preceding claims, characterised by
(a) an additional Pro codon upstream of the DNA sequence coding for PTH or
(b) a Cys codon in place of the Ser(1) codon of the PTH gene or
(c) an additional Met codon upstream of the DNA sequence coding for PTH and two Leu codons in place of the Met(8) and the Met(18) codons of the PTH gene.

4. An expression vector according to claim 1 having the following features:
(a) P_{R}-promoter (Fig. 1A1);
(b) optional deletion of the DNA region between the two HpaI cleavage sites (Fig. 1A1), which both lie in the reading frame and are downstream of the codon for amino acid 178 and for amino acid 386, respectively, of the Cro/β-galactosidase protein (lacZ gene);
(c) EcoRV cleavage site immediately downstream of the codon for amino acid 407 (Asp) of the Cro/β-galactosidase protein (lacZ gene);
(d1) PTH gene inserted into the EcoRV cleavage site according to (c) at the 5' end (Fig. 1A1), which PTH gene has the following features:
(d2) the Ile codon ATA has optionally been replaced by ATT (Fig. 1B);
(d3) the DNA sequence as far as the NsiI cleavage site of the PTH gene (Fig. B) has been chemically synthesised;
(d4) the remaining portion of the PTH gene (as far as the XbaI cleavage site; Fig. 1A1) originates from a PTH gene that has been isolated from a genomic cosmid gene bank, that portion having been inserted into the XbaI cleavage site at the 3' end; and further
(d5) an additional Pro codon (Fig. 1B1) upstream of the DNA sequence coding for PTH; and
(d6) a new BamHI cleavage site (Fig. 1A1 and 1A2) that has been produced at the former EcoRV cleavage site according to (c) (or fusion site) and has optionally been filled up (Fig. 1A1), the DNA sequence (228 amino acids) between the first HpaI cleavage site (see (b); Fig. 1A1) and the BamHI cleavage site (Fig. 1A1 and 1A3) having been deleted.

5. An expression vector according to claim 1 having the following features:
(a) P_{R}-promoter (Fig. 1A1);
(b) optional deletion of the DNA region between the two HpaI cleavage sites (Fig. 1A1), which both lie in the reading frame and are downstream of the codon for amino acid 178 and for amino acid 386, respectively, of the Cro/β-galactosidase protein (lacZ gene);
(c) EcoRV cleavage site immediately downstream of the codon for amino acid 407 (Asp) of the Cro/β-galactosidase protein (lacZ gene);
(d1) PTH gene inserted into the EcoRV cleavage site according to (c) at the 5' end (Fig. 1A1), which PTH gene has the following features:
(d2) the Ile codon ATA has optionally been replaced by ATT (Fig. 1B);
(d3) the DNA sequence as far as the NsiI cleavage site of the PTH gene (Fig. B) has been chemically synthesised;
(d4) the remaining portion of the PTH gene (as far as the XbaI cleavage site; Fig. 1A1) originates from a PTH gene that has been isolated from a genomic cosmid gene bank, that portion having been inserted into the XbaI cleavage site at the 3' end; and further
(d5) a Cys codon in place of the Ser(1) codon of the PTH gene (Fig. 1B2).

6. An expression vector according to claim 1 having the following features:
(a) P_{R}-promoter (Fig. 1A1);
(b) optional deletion of the DNA region between the two HpaI cleavage sites (Fig. 1A1), which both lie in the reading frame and are downstream of the codon for amino acid 178 and for amino acid 386, respectively, of the Cro/β-galactosidase protein (lacZ gene);
(c) EcoRV cleavage site immediately downstream of the codon for amino acid 407 (Asp) of the Cro/β-galactosidase protein (lacZ gene);
(d1) PTH gene inserted into the EcoRV cleavage site according to (c) at the 5' end (Fig. 1A1), which PTH gene has the following features:
(d2) the Ile codon ATA has optionally been replaced by ATT (Fig. 1B);
(d3) the DNA sequence as far as the NsiI cleavage site of the PTH gene (Fig. B) has been chemically synthesised;
(d4) the remaining portion of the PTH gene (as far as the XbaI cleavage site; Fig. 1A1) originates from a PTH gene that has been isolated from a genomic cosmid gene bank, that portion having been inserted into the XbaI cleavage site at the 3' end; and further
(d5) an additional Met codon (Fig. 1B3) upstream of the DNA sequence coding for PTH; and
(d6) two Leu codons in place of the Met(8) and the Met(18) codons of the PTH gene (Fig. 1B3).

7. An expression vector according to any one of the preceding claims, characterised in that the PTH gene is the pPTH, bPTH or hPTH gene.

8. A method of obtaining a Cro/β-galactosidase/PTH fusion protein, characterised in that the fusion protein is expressed in E. coli with the aid of an expression vector according to any one of the preceding claims, is extracted and, optionally, is isolated.

9. A method of obtaining PTH, characterised in that a Cro/β-galactosidase/PTH fusion protein is expressed in E. coli with the aid of the expression vector according to claim 3(a) or 4 and the fusion protein obtained in that manner and by extraction is cleaved under acid conditions, the N-terminal proline residue is removed and PTH is purified and obtained in a manner known per se, or in that the fusion protein obtained in accordance with claim 8 with the aid of the expression vector according to claim 3(a) or 4 and by extraction is cleaved under acid conditions, the N-terminal proline residue is removed and PTH is purified and obtained in a manner known per se.

10. A method of obtaining PTH, characterised in that a Cro/β-galactosidase/PTH fusion protein is expressed in E. coli with the aid of an expression vector according to claim 3(b) or 5 and the fusion protein obtained in that manner and by extraction
(a) is oxidised to the disulphide and cleaved with cyanide, a cyclised cysteine residue being produced at the N-terminus, or
(b) after reversibly protecting the methionine residues and alkylating the Cys, is cleaved with cyanogen bromide, the cysteine residue being converted into the natural serine residue,
and PTH is purified and obtained in a manner known per se, or
in that the fusion protein obtained in accordance with claim 8 with the aid of the expression vector according to claim 3(b) or 5 and by extraction
(a) is oxidised to the disulphide and cleaved with cyanide, a cyclised cysteine residue being produced at the N-terminus, or
(b) after reversibly protecting the methionine residues and alkylating the Cys, is cleaved with cyanogen bromide, the cysteine residue being converted into the natural serine residue,
and PTH is purified and obtained in a manner known per se.

11. A method of obtaining PTH, characterised in that a Cro/β-galactosidase/PTH fusion protein is expressed in E. coli with the aid of the expression vector according to claim 3(c) or 6 and PTH is split off, using cyanogen bromide, from the fusion protein obtained in that manner and by extraction, and is purified and obtained in a manner known per se, or
in that PTH is split off, using cyanogen bromide, from the fusion protein obtained in accordance with claim 8 with the aid of the expression vector according to claim 3(c) or 6 and by extraction, and is purified and obtained in a manner known per se.

12. A method according to claim 9, 10 or 11, characterised in that the PTH released is purified in the form of an aqueous buffered urea-containing solution with a stepped gradient over carboxymethylcellulose.

## Revendications

1. Vecteur d'expression pour *E. coli,* comportant (suivant le sens de transcription) :
- un gène de résistance à un antibiotique,
- le gène d'une protéine hybride et
- le gène d'une protéine voulue,
le vecteur d'expression étant caractérisé en ce qu'il est constitué par pEX2 comportant les modifications suivantes :
- une délétion réalisée, au minimum, dans la zone allant du site de restriction EcoRV jusqu'au site de restriction XbaI et, au maximum, dans la zone allant du site de restriction HpaI (en aval et au voisinage du site de restriction HindIII) jusqu'au site de restriction XbaI et
- l'insertion du gène PTH (en tant que gène de la protéine voulue) avant le site de restriction XbaI.

2. Vecteur d'expression suivant la revendication 1, caractérisé en ce que l'ATA du codon Ile du gène PTH est remplacé par ATT.

3. Vecteur d'expression suivant l'une des revendications précédentes, caractérisé par :
(a) un Pro-codon supplémentaire avant la séquence d'ADN codant pour la PTH ou
(b) un codon Cys à la place du codon Ser(1) du gène PTH ou
(c) un codon Met supplémentaire avant la séquence d'ADN codant pour la PTH et deux codons Leu à la place du codon Met(8) et du codon Met(18) du gène PTH.

4. Vecteur d'expression suivant la revendication 1, comportant les particularités suivantes :
(a) un promoteur P_{R} (figure 1A1) ;
(b) une délétion éventuelle du fragment d'ADN situé entre les deux sites de restriction HpaI (figure 1A1) qui sont situés l'un et l'autre dans le cadre de lecture derrière le codon correspondant respectivement à l'acide aminé 178 ou à l'acide aminé 386 de la protéine Cro/β-galactosidase (gène lacZ) ;
(c) un site de restriction EcoRV directement derrière le codon correspondant à l'acide aminé 407 (Asp) de la protéine Cro/β-galactosidase (gène lacZ) ;
(d1) un gène PTH inséré du côté 5' dans le site de restriction EcoRV conforme à (c) (figure 1A1) présentant les particularités suivantes :
(d2) l'ATA du codon Ile est éventuellement remplacé par ATT (figure 1B) ;
(d3) la séquence d'ADN jusqu'au site de restriction NsiI du gène PTH (figure B) est synthétisée chimiquement ;
(d4) le reste du gène PTH (jusqu'au site de restriction XbaI ; figure 1A1) est issu d'un gène PTH qui a été isolé à partir d'une banque génomique de gènes cosmides, ce reste étant inséré du côté 3' dans le site de restriction XbaI ; et en outre
(d5) un Pro-codon supplémentaire (figure 1B1) avant la séquence d'ADN codant pour la PTH ; et
(d6) un nouveau site de restriction BamHI (figures 1A1 et 1A2) qui est produit au niveau du précédent site de restriction EcoRV conforme à (c) (ou site de fusion) et est éventuellement rempli (figure 1A1), la séquence d'ADN (228 acides aminés) faisant l'objet d'une délétion entre le premier site de restriction HpaI (voir (b) ; figure 1A1) et le site de restriction BamHI (figures 1A1 et 1A3).

5. Vecteur d'expression suivant la revendication 1, comportant les particularités suivantes :
(a) un promoteur P_{R} (figure 1A1) ;
(b) une délétion éventuelle du fragment d'ADN situé entre les deux sites de restriction HpaI (figure 1A1) qui sont situés l'un et l'autre dans le cadre de lecture derrière le codon correspondant respectivement à l'acide aminé 178 ou à l'acide aminé 386 de la protéine Cro/β-galactosidase (gène lacZ) ;
(c) un site de restriction EcoRV directement derrière le codon correspondant à l'acide aminé 407 (Asp) de la protéine Cro/β-galactosidase (gène lacZ) ;
(d1) un gène PTH inséré du côté 5' dans le site de restriction EcoRV conforme à (c) (figure 1A1) présentant les particularités suivantes :
(d2) l'ATA du codon Ile est éventuellement remplacé par ATT (figure 1B) ;
(d3) la séquence d'ADN jusqu'au site de restriction NsiI du gène PTH (figure B) est synthétisée chimiquement ;
(d4) le reste du gène PTH (jusqu'au site de restriction XbaI ; figure 1A1) est issu d'un gène PTH qui a été isolé à partir d'une banque génomique de gènes cosmides, ce reste étant inséré du côté 3' dans le site de restriction XbaI ; et en outre
(d5) un codon Cys à la place du codon Ser(1) du gène PTH (figure 1B2).

6. Vecteur d'expression suivant la revendication 1, comportant les particularités suivantes :
(a) un promoteur P_{R} (figure 1A1) ;
(b) une délétion éventuelle du fragment d'ADN situé entre les deux sites de restriction HpaI (figure 1A1) qui sont situés l'un et l'autre dans le cadre de lecture derrière le codon correspondant respectivement à l'acide aminé 178 ou à l'acide aminé 386 de la protéine Cro/β-galactosidase (gène lacZ) ;
(c) un site de restriction EcoRV directement derrière le codon correspondant à l'acide aminé 407 (Asp) de la protéine Cro/β-galactosidase (gène lacZ) ;
(d1) un gène PTH inséré du côté 5' dans le site de restriction EcoRV conforme à (c) (figure 1A1) présentant les particularités suivantes :
(d2) l'ATA du codon Ile est éventuellement remplacé par ATT (figure 1B) ;
(d3) la séquence d'ADN jusqu'au site de restriction NsiI du gène PTH (figure B) est synthétisée chimiquement ;
(d4) le reste du gène PTH (jusqu'au site de restriction XbaI ; figure 1A1) est issu d'un gène PTH qui a été isolé à partir d'une banque génomique de gènes cosmides, ce reste étant inséré du côté 3' dans le site de restriction XbaI ; et en outre
(d5) un codon Met supplémentaire (figure 1B3) avant la séquence d'ADN codant pour la PTH ; et
(d6) deux codons Leu à la place du codon Met(8) et du codon Met(18) du gène PTH (figure 1B3).

7. Vecteur d'expression suivant l'une des revendications précédentes, caractérisé par le gène pPTH, le gène bPTH ou le gène hPTH en tant que gène PTH.

8. Procédé d'obtention d'une protéine de fusion Cro/β-galactosidase/PTH, caractérisé en ce qu'on exprime la protéine de fusion dans *E. coli* au moyen d'un vecteur d'expression conforme à l'une des revendications précédentes, on l'extrait et on l'isole éventuellement.

9. Procédé d'obtention de PTH, caractérisé en ce qu'on exprime une protéine de fusion Cro/β-galactosidase/PTH dans *E. coli* au moyen du vecteur d'expression conforme à la revendication 3 (a) ou la revendication 4 et on procède à un clivage acide de la protéine de fusion obtenue de cette manière et par extraction, on sépare le reste proline N-terminal et on procède à la purification et la récupération de PTH d'une manière en soi connue,
ou en ce qu'on procède à un clivage acide de la protéine de fusion obtenue conformément à la revendication 8 au moyen du vecteur d'expression conforme à la revendication 3 (a) ou la revendication 4 et par extraction, on sépare le reste proline N-terminal et on procède à une purification et une récupération de PTH d'une manière en soi connue.

10. Procédé d'obtention de PTH, caractérisé en ce qu'on procède à l'expression d'une protéine de fusion Cro/β-galactosidase/PTH dans *E. coli* au moyen d'un vecteur d'expression conforme à la revendication 3 (b) ou la revendication 5 et on soumet la protéine de fusion obtenue de cette manière et par extraction ;
(a) à une oxydation en disulfure et à un clivage au moyen de cyanure, de sorte qu'en position N-terminale, il se forme un reste cystéine cyclisé, ou
(b) à un clivage après protection réversible des restes méthionine et alcoylation de la Cys au moyen de bromure de cyanogène, de sorte que le reste cystéine est transformé en reste sérine naturel,
et on soumet la PTH à une purification et une récupération d'une manière en soi connue, ou
en ce qu'on soumet une protéine de fusion obtenue conformément à la revendication 8 au moyen du vecteur d'expression suivant la revendication 3 (b) ou la revendication 5 et par extraction ;
(a) à une oxydation en disulfure et à un clivage au moyen de cyanure, de sorte qu'en position N-terminale, il se forme un reste cystéine cyclisé, ou
(b) à un clivage après protection réversible des restes méthionine et alcoylation de la Cys au moyen de bromure de cyanogène, de sorte que le reste cystéine est transformé en reste sérine naturel,
et on soumet la PTH à une purification et une récupération d'une manière en soi connue.

11. Procédé d'obtention de PTH, caractérisé en ce qu'on procède à l'expression d'une protéine de fusion Cro/β-galactosidase/PTH dans *E. coli* au moyen du vecteur d'expression conforme à la revendication 3 (c) ou la revendication 6, on soumet la PTH à un clivage, au moyen de bromure de cyanogène, à partir de la protéine de fusion récupérée de cette manière et par extraction, et on la soumet à une purification et une récupération d'une manière en soi connue ou
en ce qu'on procède à un clivage de la PTH, au moyen de bromure de cyanogène, à partir de la protéine de fusion récupérée conformément à la revendication 8 au moyen du vecteur d'expression conforme à la revendication 3 (c) ou la revendication 6 et par extraction, et on la soumet à une purification et une récupération d'une manière en soi connue.

12. Procédé suivant l'une des revendications 9 à 11, caractérisé en ce qu'on purifie la PTH libérée, sous la forme d'une solution aqueuse contenant de l'urée et tamponnée, avec un gradient étagé, sur de la carboxyméthylcellulose.
